# EUROPÄISCHE PATENTSCHRIFT

(11) **EP 2 831 038 B1**
(45) Veröffentlichungstag und Bekanntmachung des Hinweises auf die Patenterteilung: **19.07.2017**
(21) Anmeldenummer: 13712251.1
(22) Anmeldetag: 25.03.2013
(51) Int. Cl.: C07C 231/12, C07C 233/52, C07D 263/52, C07D 209/96

(54) **VERFAHREN ZUR HERSTELLUNG VON CIS-ALKOXYSUBSTITUIERTEN SPIROCYCLISCHEN PHENYLACETYLAMINOSÄUREESTERN UND CIS-ALKOXYSUBSTITUIERTEN SPIROCYCLISCHEN 1-H-PYRROLIDIN-2,4-DION-DERIVATEN**
METHOD FOR PRODUCING CIS-ALKOXY SUBSTITUTED CYCLIC PHENYLACETYL AMINO ACID ESTERS
PROCÉDÉ DE FABRICATION D'ESTER D'ACIDES AMINÉS DE PHÉNYLACÉTYLAMINE CYCLIQUES CIS-ALKOXY-SUBSTITUÉS

(30) Priorität: 28.03.2012 EP 12161837
(43) Veröffentlichungstag der Anmeldung: 04.02.2015
(73) Patentinhaber: Bayer Intellectual Property GmbH, 40789 Monheim (DE); Bayer CropScience AG, 40789 Monheim am Rhein (DE)
(72) Erfinder: FARIDA, Taraneh, 50259 Pulheim-Geyen (DE); MAIWALD, Berndt, 51399 Burscheid (DE); LITTMANN, Martin, 51375 Leverkusen (DE); ETZEL, Winfried, 42799 Leichlingen (DE); WARSITZ, Rafael, 45136 Essen (DE); HENCK, Nioclas, 53229 Bonn (DE); ESSER, Michael, 51379 Leverkusen (DE)
(74) Vertreter: BIP Patents
(86) Internationale Anmeldenummer: PCT/EP2013/056318
(87) Internationale Veröffentlichungsnummer: WO 2013/144101

(56) Entgegenhaltungen:
- WO-A1-2004/007448
- HARRISON H R ET AL: "USE OF MOLECULAR SIEVES IN THE METHYL ESTERIFICATION OF CARBOXYLIC ACIDS", JOURNAL OF APPLIED CHEMISTRY, SOCIETY OF CHEMICAL INDUSTRY. LONDON, GB, Bd. 75, 9. November 1968 (1968-11-09), Seite 1568, XP000925894,

## Beschreibung

Die vorliegende Erfindung betrifft ein neues Verfahren zur Herstellung von cis-alkoxysubstituierten spirocyclischen Phenylacetylaminosäureestern und cis-alkoxysubstituierten spirocyclischen 1-H-Pyrrolidin-2,4-dion-Derivaten sowie neue Intermediate bzw. Ausgangsverbindungen, welche in dem erfindungsgemäßen Verfahren durchlaufen bzw. verwendet werden. Cis-alkoxysubstituierte spirocyclische Phenylacetylaminosäureester sind wichtige Zwischenprodukte zur Synthese von insektiziden/akariziden Wirkstoffen.

Cis-alkoxysubstituierte spirocyclische Phenylacetylaminosäureester sind z.B. aus WO 04/007448 bekannt. Man erhält cis-alkoxysubstituierte spirocyclische Phenylacetylaminosäureester der Formel (I), wenn man cis-Aminosäurederivate der Formel (VI) 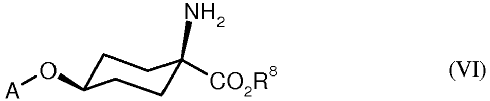 mit substituierten Phenylessigsäurehalogeniden der Formel (VII) 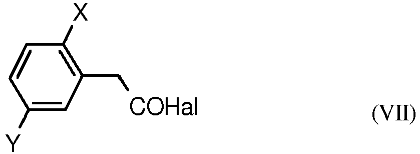 acyliert (Chem. Reviews 52, 237-416 (1953); Bhattacharya, Indian J. Chem. 6, 341-5, 1968) oder wenn man cis-Aminosäuren der Formel (VIII) 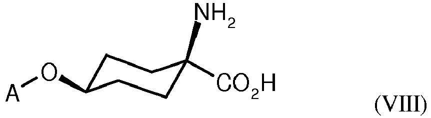 mit substituierten Phenylessigsäurehalogeniden der Formel (VII) 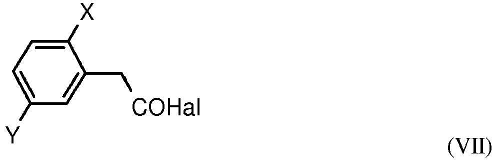 nach Schotten-Baumann acyliert (Organikum, VEB Deutscher Verlag der Wissenschaften, Berlin 1977, S. 505) und verestert (Chem. Ind. (London) 1568 (1968)).

Beim bisherigen Verfahren wird die cis-alkoxysubstituierte spirocyclische Phenylacetylaminosäure (Verbindungen der Formel (IIa)) mit Chlorbenzol in der Wärme extrahiert. Diese Lösung wird mit Methanol unter Katalyse von Schwefelsäure zu Verbindungen der Formel (I) verestert (Teilveresterung der Verbindungen der Formel (IIa)). Der nicht veresterte Teil wird zurückgeführt. Da die Verbindungen der Formel (I) bei Raumtemperatur nicht in Chlorbenzol gelöst bleiben, findet ein Lösungsmittelaustausch Chlorbenzol gegen Dimethylacetamid statt.

Nachteilig an diesem Verfahren ist insbesondere, dass die Ausbeuten unbefriedigend sind und dass der Verfahrensaufwand hoch ist. Außerdem werden die verwendeten Lösungsmittel Chlorbenzol und Dimethylacetamid (DMAC) ungern eingesetzt. Die Entsorgung des DMAC-haltigen Abwassers ist mit hohen Kosten verbunden.

Die Aufgabe der vorliegenden Erfindung besteht in der Bereitstellung eines neuen, wirtschaftlich und ökologisch günstigeren Verfahren zur selektiven Herstellung von cis-alkoxysubstituierten spirocyclischen Phenylacetylaminosäureester. Insbesondere soll das erfindungsgemäße Verfahren mit gängigen Lösungsmitteln auskommen. Toluol, Xylol, Alkane wie n-Hexan, n-Heptan, n-Octan, Ether wie Diethy-, Diisopropyl-, Dibutylether, Anisol, Methyl-tert-butylether, Methyl-tert-amylether, Glykoldimethylether, Diglykoldimethylether, Tetrahydrofuran oder Dioxan, Ketone wie Aceton, Methyl- ethyl-, Methyl-isopropyl- oder Methyl- isobutyl-keton (MIBK), Kohlenwasserstoffe wie Pentan, Hexan, Heptan, Cyclohexan, Methylcyclohexan, Benzol, halogenierte Kohlenwasserstoffe wie Methylenchlorid, Chloroform, Tetrachlorkohlenstoff, Chlorbenzol, o-Dichlorbenzol, Dichlorethan können verwendet werden. Bevorzugt verwendet werden Toluol oder Xylol. Besonders bevorzugt wird Xylol verwendet.

Es wurde nun gefunden, dass man ein Gemisch aus Verbindungen der Formel (I) 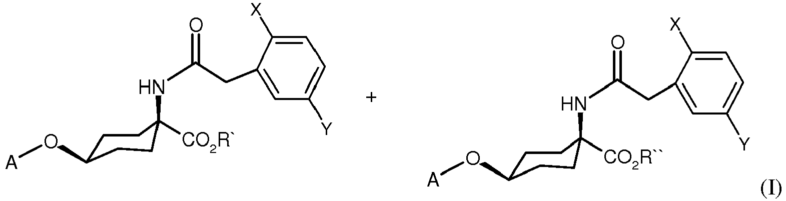 in welcher
- X: für Alkyl, Halogen, Alkoxy, Halogenalkyl oder Halogenalkoxy steht,
- Y: für Wasserstoff, Alkyl, Alkoxy, Halogen, Halogenalkyl oder Halogenalkoxy steht, wobei nur einer der Reste X oder Y für Halogenalkyl oder Halogenalkoxy stehen darf,
- A: für C₁-C₆-Alkyl steht,
- R': für Alkyl steht,

- R": für Alkyl steht,
erhält,
in dem man zunächst Verbindungen der Formel (IIa) 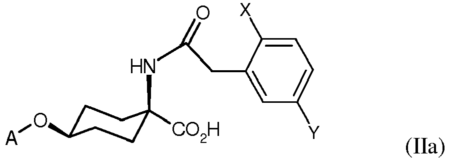 in welcher X, Y und A die oben genannten Bedeutungen haben
in Gegenwart einer Base zu Verbindungen der Formel (IIb) 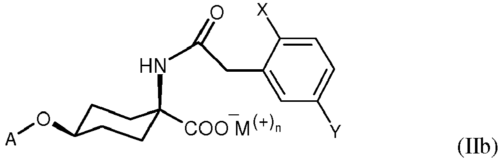
in welcher X, Y und A die oben genannten Bedeutungen haben und
M für ein Alkalimetallion, ein Ionenäquivalent eines Erdalkalimetalls, ein Ionenäquivalent Aluminium oder ein Ionenäquivalent eines Übergangsmetalls steht oder weiterhin
für ein Ammoniumion, bei dem gegebenenfalls ein, zwei, drei oder alle vier Wasserstoffatome durch gleiche oder verschiedene Reste aus den Gruppen C₁-C₅-Alkyl, C₁-C₅-Isoalkyl oder C₃-C₇-Cycloalkyl, die jeweils ein- oder mehrfach mit Fluor, Chlor, Brom, Cyano, Hydroxy substituiert oder durch ein- oder mehrere Sauerstoff- oder Schwefelatome unterbrochen sein können, ersetzt sein können, steht, oder weiterhin
für ein cyclisches sekundäres oder tertiäres aliphatisches oder heteroaliphatisches Ammoniumion, beispielsweise Morpholinium, Thiomorpholinium, Piperidinium, Pyrrolidinium, oder jeweils protoniertes 1,4-Diazabicyclo[2.2.2]octane (DABCO) oder 1,5-Diazabicyclo[4.3.0]undec-7-en (DBU), steht, oder weiterhin
für ein heterocyclisches Ammoniumkation, beispielsweise jeweils protoniertes Pyridin, 2-Methylpyridin, 3-Methylpyridin, 4-Methylpyridin, 2,4-Dimethylpyridin, 2,5-Di-methylpyridin, 2,6-Dimethylpyridin, 5-Ethyl-2-methylpyridin, Pyrrol, Imidazol, Chinolin, Chinoxalin, 1,2-Dimethylimidazol, 1,3-Dimethylimidazolium-methylsulfat, steht, oder weiterhin
für ein Sulfoniumion steht, oder weiterhin
für ein Magnesium-Halogen-Kation steht,
n für die Zahl 1 oder 2 steht
umsetzt
und mit Verbindungen der Formel (III) 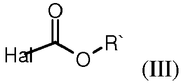 in welcher R' die oben angegebenen Bedeutungen haben
und Hal für Halogen steht,
weiter zu Verbindungen der Formel (IV) umsetzt 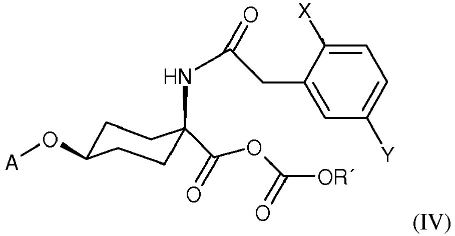 in welcher X, Y, A und R' die oben genannten Bedeutungen haben,
und die weiter zu Verbindungen der Formel (IX) 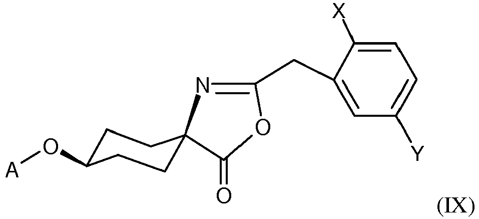 in welcher X, Y und A die oben angegebenen Bedeutungen haben,
cyclisieren, die wiederum mit Verbindungen der Formel (V)

R"-OH (V)

in welcher
R" die oben angegebenen Bedeutungen haben,
zu einem Gemisch aus Verbindungen der Formel (I) reagieren.

In den Formeln (I), (I'), (I"), (IIa), (IIb) (III), (IV), (V), (IX), (X) und (XI) steht X bevorzugt für Chlor, Brom, Methyl, Ethyl, Propyl, Trifluormethyl, Methoxy, Difluormethoxy oder Trifluormethoxy,
Y bevorzug für Wasserstoff, Chlor, Brom, Methoxy, Methyl, Ethyl, Propyl, Trifluormethyl oder Trifluormethoxy, wobei nur einer der Reste X oder Y für Trifluormethyl, Difluormethoxy oder Trifluormethoxy stehen darf,
A bevorzugt für C₁-C₆-Alkyl,
Hal bevorzugt für Chlor, Brom Fluor, Iod,
R' bevorzugt für C₁-C₆-Alkyl,
R" bevorzugt für C₁-C₆-Alkyl,
X besonders bevorzugt für Chlor, Brom, Methyl, Ethyl, Methoxy, Trifluormethyl, Trifluormethoxy oder Difluormethoxy,
Y besonders bevorzug für Chlor, Brom, Methyl, Ethyl, Propyl, Methoxy, Trifluormethyl oder Trifluormethoxy, wobei nur einer der Reste X oder Y für Trifluormethyl, Trifluormethoxy oder Difluormethoxy stehen darf,
A besonders bevorzugt für C₁-C₄-Alkyl,
Hal besonders bevorzugt für Chlor, Brom oder Fluor,
R' besonders bevorzugt für C₁-C₄-Alkyl,
R" besonders bevorzugt für C₁-C₄-Alkyl,
X ganz besonders bevorzugt für Chlor, Brom, Methyl oder Trifluormethyl, (insbsondere für Chlor, Brom oder Methyl),
Y ganz besonders bevorzugt für Chlor, Brom oder Methyl, (insbesondere für Methyl),
A ganz besonders bevorzugt für Methyl, Ethyl, Propyl, Butyl oder Isobutyl,
(insbesondere für Methyl oder Ethyl),
Hal ganz besonders bevorzugt für Chlor oder Brom,
R' ganz besonders bevorzugt für Methyl, Ethyl, Propyl, Butyl oder Isobutyl,
R" ganz besonders bevorzugt für Methyl, Ethyl, Propyl, Butyl oder Isobutyl,
X hervorgehoben für Methyl,
Y hervorgehoben für Methyl,
A hervorgehoben für Methyl,
Hal hervorgehoben für Chlor,
R' hervorgehoben für Methyl oder Ethyl (insbesondere für Ethyl),
R" hervorgehoben für Methyl oder Ethyl (insbesondere für Methyl).

In der Formel (IIb) steht
M bevorzugt für Lithium, Natrium, Kalium, Caesium, Magnesium, Calcium oder für ein Ammoniumion, bei dem gegebenenfalls ein, zwei, drei oder alle vier Wasserstoffatome durch gleiche oder verschiedene Reste aus den Gruppen C₁-C₅-Alkyl, C₁-C₅-Isoalkyl oder C₃-C₇-Cycloalkyl, die jeweils ein- oder mehrfach mit Fluor, Chlor, Brom, Cyano, Hydroxy substituiert sein können, ersetzt sein können und n für die Zahl 1 oder 2,
M besonders bevorzugt für Lithium, Natrium, Kalium, Caesium, Magnesium, Calcium und n für die Zahl 1 oder 2,
M ganz besonders bevorzugt für Lithium, Natrium, Kalium, Caesium und n für die Zahl 1,
M hervorgehoben für Natrium und n für die Zahl 1.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Iod
**Alkyl:** gesättigte, geradkettige oder verzweigte Kohlenwasserstoffreste mit 1 bis 8 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; Heptyl, Octyl.
**Halogenalkyl:** geradkettige oder verzweigte Alkylgruppen mit 1 bis 8 Kohlenstoffatomen, wobei in diesen Gruppen teilweise oder vollständig die Wasserstoffatome durch Halogenatome wie vorstehend genannt ersetzt sein können, z.B. C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2-difluorethyl, 2,2-Dichlor-2-fluorethyl, 2,2,2-Trichlorethyl, Pentafluorethyl und 1,1,1-Trifluorprop-2-yl.

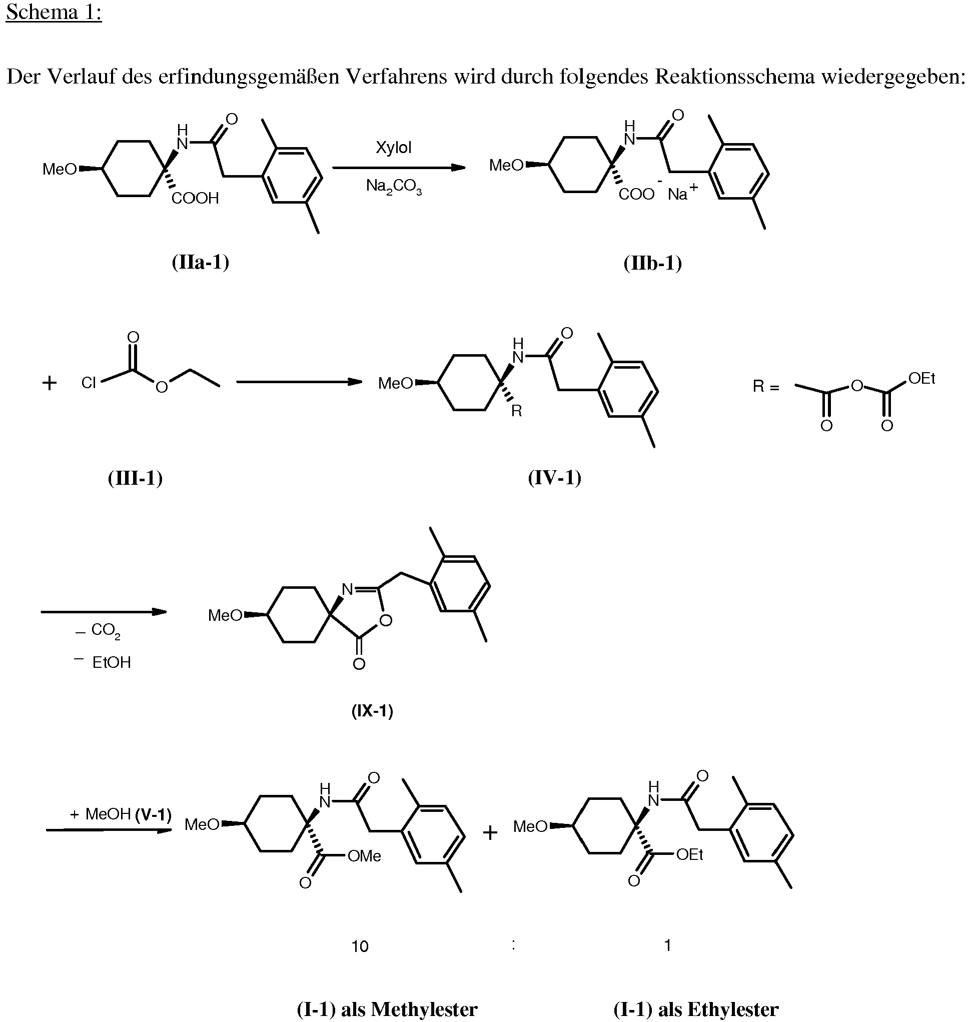

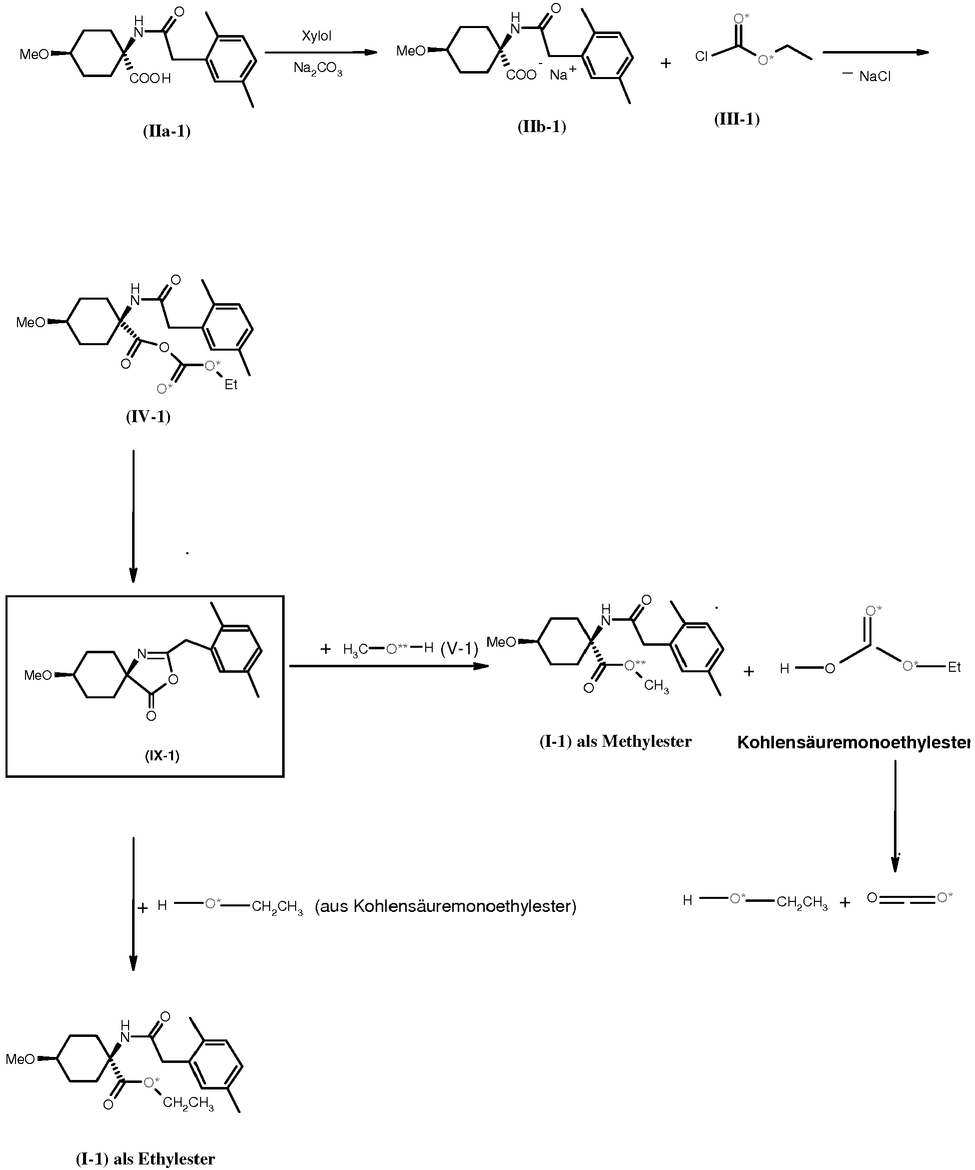

Die Indizes * und ** am Sauerstoffatom im Schema 1 sollen den Reaktionsverlauf verdeutlichen.

In Abhängigkeit der Alkoholart und Alkoholmenge (Verbindungen der Formel (V)) wird ein Estergemisch der Formel (I) erhalten (in Schema 1: Methyl- und Ethylester im Verhältnis 10:1).

Außerdem wurde gefunden, dass man Verbindungen der Formel (I') 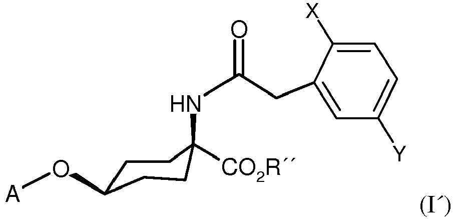 in welcher X, Y, A und R" die oben angegebenen Bedeutungen haben,
erhält,
in dem man zunächst Verbindungen der Formel (IIa) 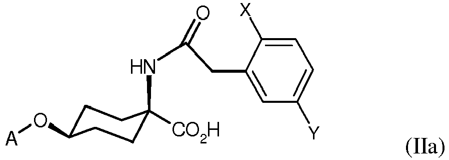 in welcher X, Y und A die oben genannten Bedeutungen haben
in Gegenwart einer Base zu Verbindungen der Formel (IIb) 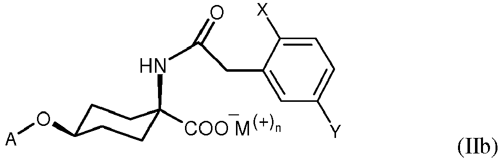 in welcher X, Y, A, M und n die oben genannten Bedeutungen haben
umsetzt
und mit Verbindungen der Formel (III) 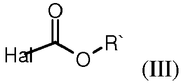 in welcher R' die oben angegebenen Bedeutungen haben
und Hal für Halogen steht,
weiter zu Verbindungen der Formel (IV) umsetzt 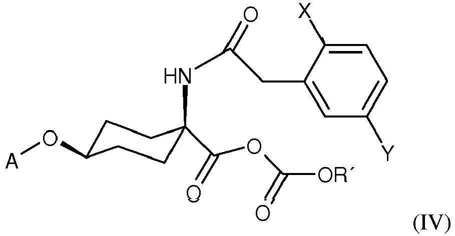 in welcher X, Y, A und R' die oben genannten Bedeutungen haben
und die weiter zu Verbindungen der Formel (IX) 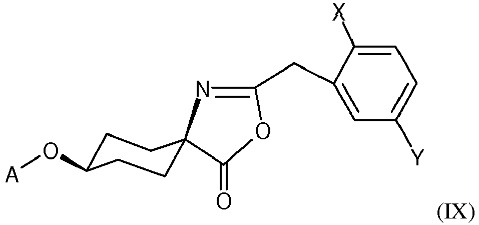 in welcher X, Y und A die oben angegebenen Bedeutungen haben, cyclisieren,
die wiederum mit Verbindungen der Formel (V)

R"-OH (V)

in welcher
R" die oben angegebenen Bedeutungen haben,
zu Verbindungen der Formel (I') reagieren, wobei R' = R".

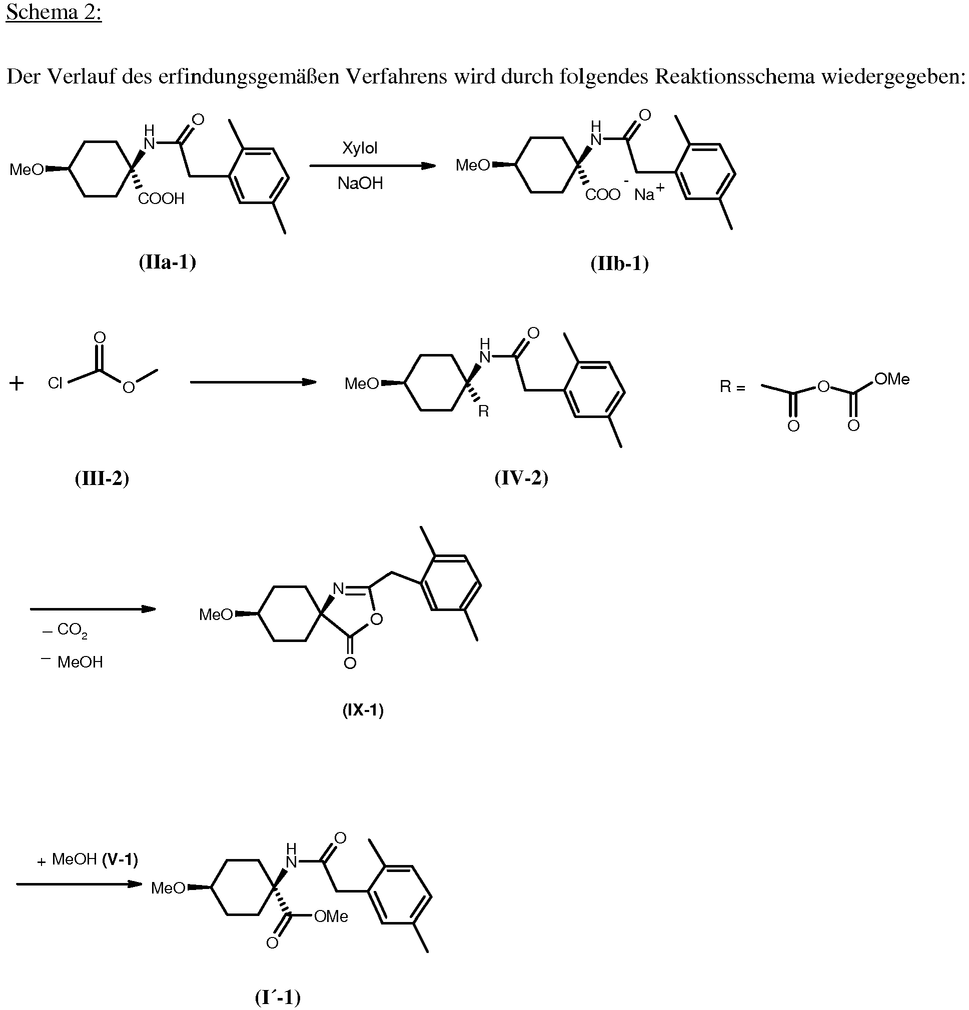

Es ist bekannt, dass man die Verbindungen der Formel (I) bzw. (I') in Gegenwart eines Verdünnungsmittels und in Gegenwart einer Base intramolekular zu cis-alkoxysubstituierten spirocyclischen 1-H-Pyrrolidin-2,4-dion-Derivaten der Formel (X) 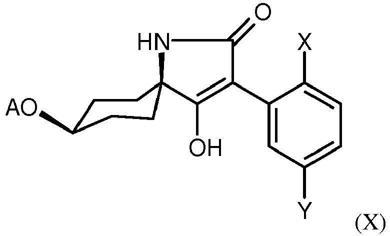 in welcher
A, X und Y die oben angegebenen Bedeutungen haben,
kondensieren kann (WO 04/007448).

Jetzt wurde gefunden, dass man die Verbindungen der Formel (X) 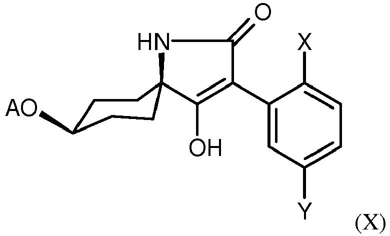 in welcher
A, X und Y die oben angegebenen Bedeutungen haben,
in einer Eintopfreaktion erhält,
in dem man Verbindungen der Formel (IIa) 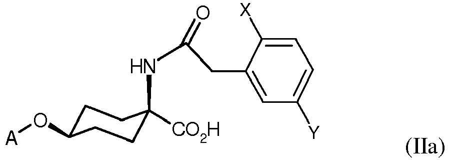 in welcher X, Y und A die oben genannten Bedeutungen haben,
in Gegenwart einer Base
zu Verbindungen der Formel (IIb) 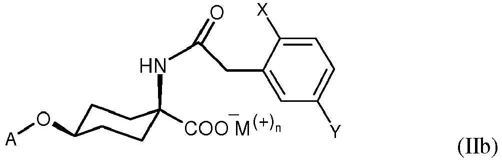 in welcher X, Y, A, M und n die oben genannten Bedeutungen haben
umsetzt
und mit Verbindungen der Formel (III) 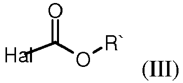 in welcher R' die oben angegebenen Bedeutungen haben und Hal für Halogen steht,
in Gegenwart einer starken Base der Formel (XI)

ZLR" (XI)

wobei
Z für ein Alkalimetallion (bevorzugt für Lithium, Natrium oder Kalium, besonders bevorzugt für Natrium oder Kalium, ganz besonders bevorzugt für Natrium) steht,
L für Sauerstoff oder Schwefel (bevorzugt für Sauerstoff) steht,
R " die oben angegebenen Bedeutungen hat,
bevorzugt werden z.Bsp. Alkoholate oder Thioalkoholate, die sowohl als Feststoff als auch als Lösung, eingesetzt werden können, beispielsweise NaOMe fest oder als Lösung in Methanol, NaOEt fest oder als Lösung in Ethanol, NaSMe fest oder als Lösung in Methanol, NaSEt fest oder als Lösung in Ethanol) umsetzt.

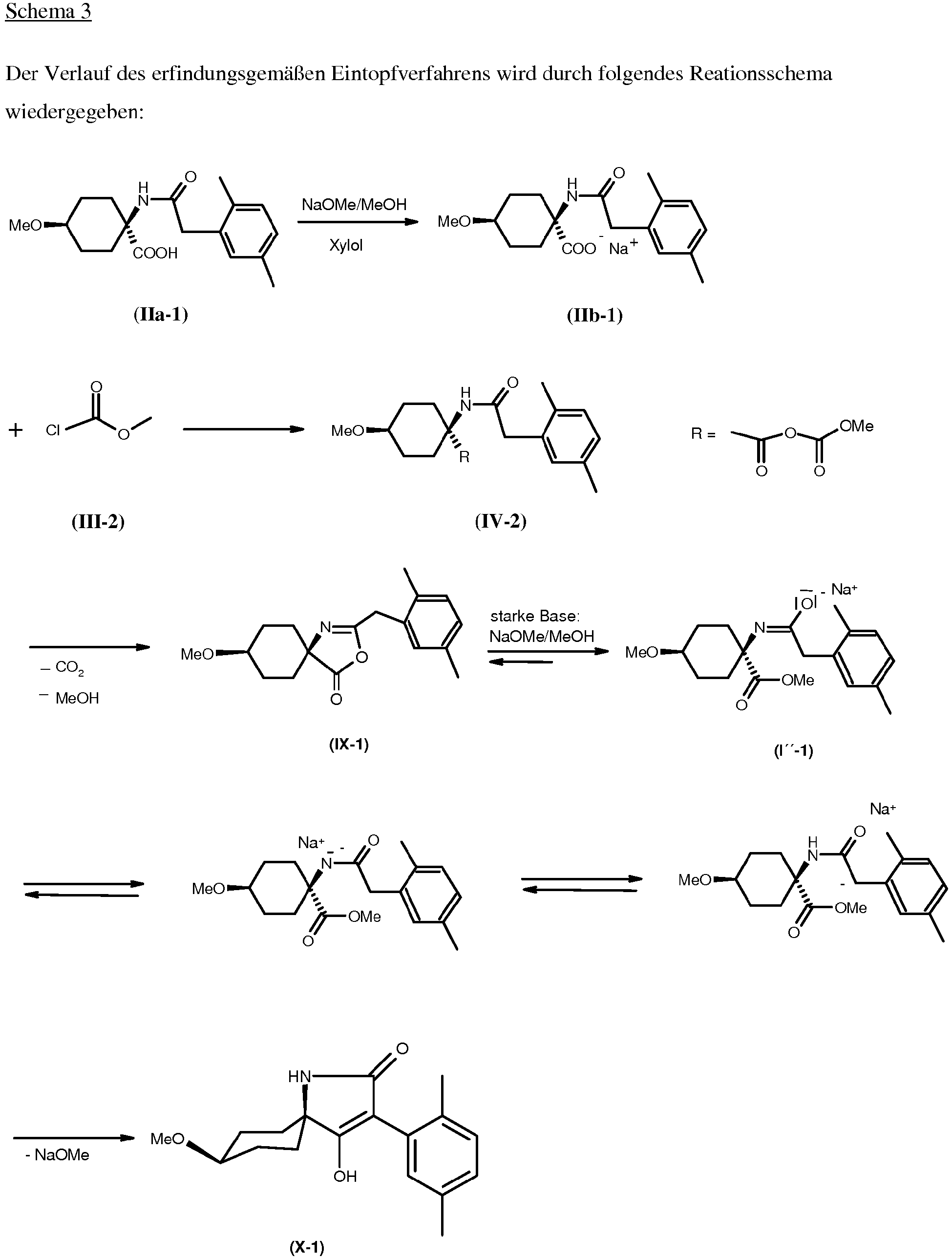

Überraschenderweise können nach dem erfindungsgemäßen Verfahren die Verbindungen der Formel (X) auf einfachere Weise, in einem Eintopfverfahren, ohne Isolierung der Zwischenstufen, in höherer Reinheit und in besserer Ausbeute hergestellt werden.

Die Verbindungen der Formeln (I') und (I) sind bekannt (WO 04/007448) oder lassen sich nach den dort beschriebenen Verfahren herstellen.

Die Verbindungen der Formel (I") sind bekannt (WO 04/007448) oder lassen sich nach den dort beschriebenen Verfahren herstellen.

Die Verbindungen der Formel (IIa) sind bekannt (WO 04/007448) oder lassen sich nach den dort beschriebenen Verfahren herstellen.

Die Verbindungen der Formel (IIb) sind neu.

Die Verbindungen der Formel (III) sind kommerziell erhältlich.

Die Verbindungen der Formel (IV) sind neu.

Die Verbindungen der Formel (V) sind kommerziell erhältlich.

Die Verbindungen der Formel (IX) sind neu.

Die Verbindungen der Formel (X) sind bekannt (WO 04/007448) oder lassen sich nach den dort beschriebenen Verfahren herstellen.

Die Verbindungen der Formel (XI) sind kommerziell erhältlich.

Bedingt durch das cis/trans Isomerenverhältnis der Verbindungen der Formel (IIa), die in das Herstellverfahren eingesetzt werden, fallen die Verbindungen der Formeln (I) bzw. (I') bzw. (I"), (IIb), (IV), (IX) und (X) in Form cis/trans Isomerengemische an, wobei in dem erfindungsgemäßen Verfahren hauptsächlich cis-Isomer entsteht.

Das Verfahren ist dadurch gekennzeichnet, dass man Verbindungen der Formel (IIa) mit hohem cis-Isomerenanteil in Gegenwart einer Base und in Gegenwart von Lösungsmitteln zum entsprechenden Salz der Formel (IIb) umsetzt. Nach azeotroper Trocknung des Reaktionsgemisches werden die Verbindungen der Formel (IIb) mit Verbindungen der Formel (III) zu Zwischenverbindungen der Formel (IV) umgesetzt. Diese cyclisieren zu Verbindungen der Formel (IX).

Die Verbindungen der Formel (IX) können in zwei tautomeren Formen vorliegen:

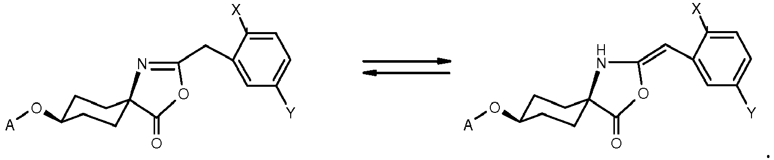

Die Verbindungen können sowohl als Gemische als auch in Form ihrer reinen Tautomeren vorliegen. Gemische lassen sich gegebenenfalls durch physikalische Methoden trennen, beispielsweise durch chromatographische Methoden.

Aus Gründen der besseren Übersichtlichkeit wird im Folgenden jeweils nur eines der möglichen Tautomeren aufgeführt. Das schließt nicht aus, dass die Verbindungen gegebenenfalls in Form der Tautomerengemische oder in der jeweils anderen tautomeren Form vorliegen können.

Die Verbindungen der Formel (IX) werden in Gegenwart eines Alkohols der Formel (V) zu Verbindungen der Formel (I) bzw. (I') umgesetzt.

Die Reaktionstemperatur zur Herstellung der Verbindungen der Formel (IIb) kann bei der Durchführung des erfindungsgemäßen Verfahrens variiert werden. Im Allgemeinen arbeitet man bei Temperaturen zwischen 20 °C und 110 °C, vorzugsweise zwischen 80 °C und 85 °C. Bevorzugt ist auch eine Temperatur von 25 °C. Ebenfalls bevorzugt ist eine Temperatur von 70°C.

Als Base können Alkoholate sowohl als Feststoff als auch als Lösung eingesetzt werden. Z. Bsp. NaOMe fest oder als Lösung in Methanol, NaOEt fest oder NaOEt als Lösung, Natriumhydrogencarbonat, Natrium- oder Kaliumhydroxid, Erdalkalihydroxide wie z. B. Calciumhydroxid, Alkalicarbonate oder - alkoholate wie Natrium- oder Kaliumcarbonat, Natrium- oder Kalium-tert-butylat. Bei den genannten Basen kann Natrium durch Kalium ersetzt werden. Bevorzugt wird Natriumcarbonat verwendet. Bevorzugt ist auch Natriummethylat in Methanol 30 %ig. Ebenfalls bevorzugt ist Natriumhydroxid.

Als Lösungsmittel können Toluol, Xylol, Alkane wie n-Hexan, n-Heptan, n-Octan, Ether wie Diethy-, Diisopropyl-, Dibutylether, Anisol, Methyl-tert-butylether, Methyl-tert-amylether, Glykoldimethylether, Diglykoldimethylether, Tetrahydrofuran oder Dioxan, Ketone wie Aceton, Methyl- ethyl-, Methyl-isopropyl- oder Methyl- isobutyl-keton (MIBK), Kohlenwasserstoffe wie Pentan, Hexan, Heptan, Cyclohexan, Methylcyclohexan, Benzol, halogenierte Kohlenwasserstoffe wie Methylenchlorid, Chloroform, Tetrachlorkohlenstoff, Chlorbenzol, o-Dichlorbenzol, Dichlorethan verwendet werden. Bevorzugt verwendet werden Toluol oder Xylol. Besonders bevorzugt wird Xylol verwendet.

Die Reaktionstemperatur zur Herstellung der Verbindungen der Formel (IV) kann bei der Durchführung des erfindungsgemäßen Verfahrens variiert werden. Im Allgemeinen arbeitet man bei Temperaturen zwischen 20 °C und 100 °C, vorzugsweise zwischen 65 °C und 70 °C, bevorzugt wird auch 70 °C.

Bei der Durchführung des erfindungsgemäßen Verfahrens setzt man die Reaktionskomponenten der Formel (III) im Allgemeinen in äquimolaren bis etwa doppeltäquimolaren Mengen ein.

Das Eintopfverfahren ist dadurch gekennzeichnet, dass man Verbindungen der Formel (IIa) mit hohem cis-Isomerenanteil in Gegenwart einer Base und in Gegenwart von Lösungsmitteln zum entsprechenden Salz der Formel (IIb) umsetzt, das mit Verbindungen der Formel (III) zu Verbindungen der Formel (IV) reagiert. Dabei entstehen die Verbindungen der Formel (IX) durch Cyclisierung, die mit einer starken Base der Formel (XI) (nucleophile Addition) zu Verbindungen der Formel (I") 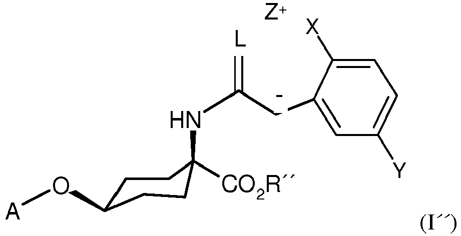 in welcher X, Y, A, L, Z und R" die oben angegebenen Bedeutungen haben, aufgespalten werden und zu Verbindungen der Formel (X) cyclisieren.
Weiterhin ist es möglich, dass die Verbindungen der Formel (IV) in Gegenwart einer starken Base direkt zu Verbindungen der Formel (X) reagieren.

Die Verbindungen der Formel (I") können in folgenden tautomeren Formen auftreten:

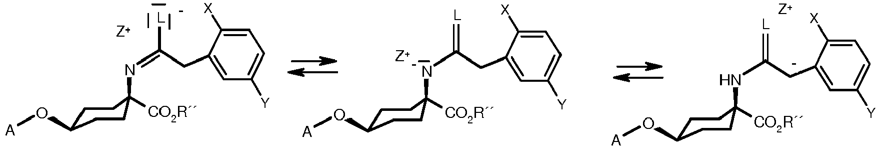

Die Verbindungen können sowohl als Gemische als auch in Form ihrer reinen Tautomeren vorliegen. Gemische lassen sich gegebenenfalls durch physikalische Methoden trennen, beispielsweise durch chromatographische Methoden.

Aus Gründen der besseren Übersichtlichkeit wird im Folgenden jeweils nur eines der möglichen Tautomeren aufgeführt. Das schließt nicht aus, dass die Verbindungen gegebenenfalls in Form der Tautomerengemische oder in der jeweils anderen tautomeren Form vorliegen können.

Die Reaktionstemperatur zur Herstellung der Verbindungen der Formel (X) ausgehend von Verbindungen der Formel (II a) kann bei der Durchführung des erfindungsgemäßen Eintopfverfahrens variiert werden. Im Allgemeinen arbeitet man bei Temperaturen zwischen 20 °C und 110 °C, vorzugsweise zwischen 60 °C und 85 °C. Bevorzugt ist eine Temperatur von 60 °C.

Als Base können die o.g. Verbindungen eingesetzt werden.

Als starke Base der Formel (XI) können z.Bsp. Alkoholate oder Thioalkoholate sowohl als Feststoff als auch als Lösung eingesetzt werden. Z.Bsp. NaOMe fest oder als Lösung in Methanol, NaOEt fest oder als Lösung in Ethanol, NaSMe fest oder als Lösung in Methanol, NaSEt fest oder als Lösung in Ethanol. Bevorzugt wird NaOMe als Lösung in Methanol.

Als Lösungsmittel können Toluol, Xylol, Alkane wie n-Hexan, n-Heptan, n-Octan, Ether wie Diethy-, Diisopropyl-, Dibutylether, Anisol, Methyl-tert-butylether, Methyl-tert-amylether, Glykoldimethylether, Diglykoldimethylether, Tetrahydrofuran oder Dioxan, Ketone wie Aceton, Methyl- ethyl-, Methyl-isopropyl- oder Methyl- isobutyl-keton (MIBK), Kohlenwasserstoffe wie Pentan, Hexan, Heptan, Cyclohexan, Methylcyclohexan, Benzol, halogenierte Kohlenwasserstoffe wie Methylenchlorid, Chloroform, Tetrachlorkohlenstoff, Chlorbenzol, o-Dichlorbenzol, Dichlorethan verwendet werden. Bevorzugt verwendet werden Toluol oder Xylol. Besonders bevorzugt wird Xylol verwendet.

Bei der Durchführung des erfindungsgemäßen Eintopfverfahrens setzt man die Reaktionskomponenten der Formel (III) im Allgemeinen in äquimolaren bis etwa doppeltäquimolaren Mengen ein.

### Herstellungsbeispiele:

### Beispiel 1 (vollständige Veresterung)

514 g (0,59 mol) cis-N-[(2,5-Dimethyl)-phenylacetyl]-1-amino-4-methoxy-cyclohexan-carbonsäure (IIa-1) (37 %ig in Xylol) werden bei 80-85 °C mit 25,7 g (0,24 mol) Natriumcarbonat 99 %ig versetzt. Das Wasser wird durch azeotrope Destillation entfernt. Bei 65 °C werden 100 g (3,12 mol) Methanol zugegeben. 80,5 g (0,72 mol) Chlorameisensäureethylester (III-1) 97 %ig werden in ca. 2 Stunden bei 65-70 °C eindosiert, wobei Kohlenstoffdioxid als Abgas entsteht. Die Reaktionsmischung wird weitere 2 Stunden bei 70 °C nachgerührt. Bei 300 mbar und 70 °C werden die Alkohole (Ethanol und Methanol) entfernt. Nach Zugabe von 150 g (8,33 mol) Wasser bei 70 °C wird die untere wässrige Phase abgetrennt. Es werden 100 g (5,56 mol) Wasser und 7 g (0,08 mol) Natriumhydrogencarbonat bei 70 °C zugegeben und die untere wässrige Phase getrennt. Nach azeotroper Trocknung erhält man das Zielprodukt (1-1) als Gemisch (Methyl- und Ethylester) in Xylol. Bei Raumtemperatur kann das Produkt durch Filtration und Trocknung isoliert werden. Im Gemisch Xylol/Methanol bleibt das Produkt (1-1) in Lösung. Die Ausbeute beträgt 98 % d. Th. bezogen auf die Verbindung der Formel (IIa-1).

### Beispiel 2 (vollständige Veresterung)

496 g (0,61 mol) cis-N-[(2,5-Dimethyl)-phenylacetyl]-1-amino-4-methoxy-cyclohexan-carbonsäure (IIa-1) (39 %ig in Xylol) werden bei 25 °C mit 113,5 g (0,63 mol) Natriummethylat in Methanol 30 %ig versetzt. 87 g (0,78 mol) Chlorameisensäureethylester (III-1) 97 %ig werden in 2 Stunden bei 65-70 °C eindosiert. Die Reaktionsmischung wird weitere 2 Stunden bei 70 °C nachgerührt. Bei 300 mbar und 70 °C werden die Alkohole (Ethanol und Methanol) entfernt. Nach Zugabe von 150 g (8,33 mol) Wasser bei 70 °C wird die untere wässrige Phase abgetrennt. Es werden 100 g (5,56 mol) Wasser und 5 g (0,06 mol) Natriumhydrogencarbonat bei 70 °C zugegeben und die untere wässrige Phase abgetrennt. Nach azeotroper Trocknung erhält man das Zielprodukt (1-1) als Gemisch (Methyl- und Ethylester) in Xylol. Bei Raumtemperatur kann das Produkt durch Filtration und Trocknung als Feststoff isoliert werden. Im Gemisch Xylol/Methanol bleibt das Produkt (1-1) in Lösung. Die Ausbeute beträgt 98 % d. Th. bezogen auf die Verbindung der Formel (IIa-1).

### Beispiel 3 (Teil Veresterung)

0,59 mol der Verbindung der Formel IIa-1 werden in 566 g (4,90 mol) Chlorbenzol vorgelegt. 71,6 g (2,24 mol) Methanol sowie 7,1 g (0,07 mol) Schwefelsäure 96 %ig werden zur Veresterung zudosiert. Das Gemisch wird auf 70 °C erwärmt und 4 Stunden bei 70 °C nachgerührt. Anschließend wird auf 40 °C abgekühlt und 25,4 g (0,30 mol) Natriumhydrogencarbonat zugegeben, wobei CO₂ frei wird. Das Reaktionsgemisch wird mit 368,9 g Wasser versetzt und ca. eine halbe Stunde bei 60 °C nachgerührt. Die untere Produktphase (Methylester der Formel (I-1)) in Chlorbenzol wird abgetrennt. Da die Verbindung der Formel (1-1) bei Raumtemperatur nicht gelöst bleibt, findet ein Lösungsmittelaustausch Chlorbenzol gegen Dimethylacetamid statt.

Die obere wässrige Phase enthält die nicht veresterte Verbindung der Formel (IIa-1), die zurückgeführt und verestert wird. Die Ausbeute beträgt 89 % d. Th. bezogen auf die Verbindung der Formel (IIa-1).

### Beispiel 4 (vollständige Veresterung)

400 g (0,5 mol) cis-N-[(2,5-Dimethyl)-phenylacetyl]-1-amino-4-methoxy-cyclohexan-carbonsäure (IIa-1) (39,9 %ig in Xylol) werden bei 70 °C mit 40 g (0,32mol) Natronlauge 32 %ig versetzt. Das Wasser wird durch azeotrope Destillation entfernt. Bei 70 °C werden 31 g (0,95 mol) Methanol zugegeben. 52 g (0,55 mol) Chlorameisensäuremethylester (III-2) 99 %ig werden in ca. 2 Stunden bei 70 °C eindosiert, wobei Kohlenstoffdioxid als Abgas entsteht. Die Reaktionsmischung wird weitere 2 Stunden bei 70 °C nachgerührt. Bei 100 mbar und 70 °C wird der Alkohol (Methanol) entfernt. Nach Zugabe von 120 g (6,7 mol) Wasser bei 70 °C wird die untere wässrige Phase abgetrennt. Es werden 80 g (4,4 mol) Wasser und 45 g (0,36 mol) Natronlauge 32 %ig bei 70 °C zugegeben und die untere wässrige Phase getrennt. Nach azeotroper Trocknung erhält man das Zielprodukt (I'-1) als (Methylester) in Xylol. Bei 20 °C kann das Produkt durch Filtration und Trocknung isoliert werden. Im Gemisch Xylol/Methanol bleibt das Produkt in Lösung. Die Ausbeute beträgt 92 % d. Th. bezogen auf die Verbindung der Formel (IIa-1).

### Beispiel 5

530,037 g (0,500 mol) der Verbindung der Formel IIa-1 30,1 %ig in Xylol wird mit 27,012 g (0,150 mol) NaOMe in Methanol 30 %ig versetzt. Das Methanol wird im Vakuum bei 60 °C und 100 mbar abdestilliert. Bei 60 °C werden in 1-2 Std. 52,498 g (0,550 mol) Chlorameisensäuremethylester (III-2) 99 %ig zu getropft und 0,5 Std. bei 60 °C nachgerührt. Die Verbindung der Formel (IX-1) wird als Rohprodukt aus dem Reaktionsgemisch erhalten.

¹³C-NMR (CD₃CN, 1.30 ppm): δ=181.9 (O-C=O), 162.8 (N=C-O), 135.1 (PheC-CH₂), 133.1 (PheC-CH₃), 132.0 (Phe-C-H), 77.6 (H-C-OMe), 68.7 (=N-C-C=O), 56.3 (O-CH₃), 34.5 (N=C-CH2-Phe), 32.2 (CH₂-CH₂-C-N=), 27.6 (MeO-CH-CH₂), 20.3 (Phe-CH₃) ppm.

Vier Signale der Phenylgruppe können nicht zugeordnet werden aufgrund starker Überlappung mit anderen Komponenten des Reaktionsgemischs.

### Beispiel 6

530,037 g (0,500 mol) der Verbindung der Formel IIa-1 30,1 %ig in Xylol wird mit 27,012 g (0,150 mol) NaOMe in Methanol 30 %ig versetzt. Das Methanol wird im Vakuum bei 60 °C und 100 mbar abdestilliert. Bei 60 °C werden in 1-2 Std. 52,498 g (0,550 mol) Chlorameisensäuremethylester (III-2) 99 %ig zu getropft und 0,5 Std. bei 60 °C nachgerührt.

Der überschüssige Chlorameisensäuremethylester und Reste des Alkohols werden destilliert. Anschließend werden 99 g (0,550 mol) NaOMe in MeOH 30 %ig zudosiert und 1 Std. bei 60 °C nachgerührt. Das Enol (X-1) wird dabei erhalten.

## Patentansprüche

1. Verfahren zur Herstellung von Verbindungen der Formel (I) 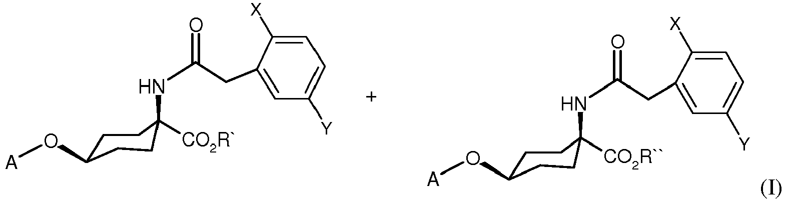 in welcher
X für Alkyl, Halogen, Alkoxy, Halogenalkyl oder Halogenalkoxy steht,
Y für Wasserstoff, Alkyl, Alkoxy, Halogen, Halogenalkyl oder Halogenalkoxy steht, wobei nur einer der Reste X oder Y für Halogenalkyl oder Halogenalkoxy stehen darf,
A für C₁-C₆-Alkyl steht,
R' für Alkyl steht,
R" für Alkyl steht,
**dadurch gekennzeichnet, dass** man zunächst
Verbindungen der Formel (IIa) 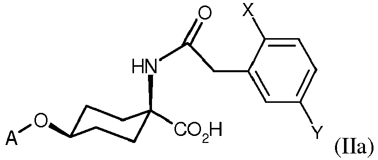 in welcher X, Y und A die oben genannten Bedeutungen haben
in Gegenwart einer Base zu Verbindungen der Formel (IIb) 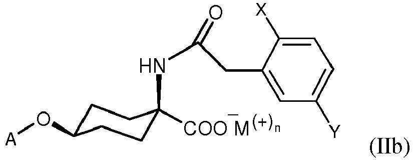 in welcher X, Y und A die oben genannten Bedeutungen haben und
M für ein Alkalimetallion, ein Ionenäquivalent eines Erdalkalimetalls, ein Ionenäquivalent Aluminium oder ein Ionenäquivalent eines Übergangsmetalls steht oder weiterhin für ein Ammoniumion, bei dem gegebenenfalls ein, zwei, drei oder alle vier Wasserstoffatome durch gleiche oder verschiedene Reste aus den Gruppen C₁-C₅-Alkyl, C₁-C₅-Isoalkyl oder C₃-C₇-Cycloalkyl, die jeweils ein- oder mehrfach mit Fluor, Chlor, Brom, Cyano, Hydroxy substituiert oder durch ein- oder mehrere Sauerstoff- oder Schwefelatome unterbrochen sein können, ersetzt sein können, steht, oder weiterhin
für ein cyclisches sekundäres oder tertiäres aliphatisches oder heteroaliphatisches Ammoniumion, beispielsweise Morpholinium, Thiomorpholinium, Piperidinium, Pyrrolidinium, oder jeweils protoniertes 1,4-Diazabicyclo[2.2.2]octane (DABCO) oder 1,5-Diazabicyclo[4.3.0]undec-7-en (DBU), steht, oder weiterhin
für ein heterocyclisches Ammoniumkation, beispielsweise jeweils protoniertes Pyridin, 2-Methylpyridin, 3-Methylpyridin, 4-Methylpyridin, 2,4-Dimethylpyridin, 2,5-Di-methylpyridin, 2,6-Dimethylpyridin, 5-Ethyl-2-methylpyridin, Pyrrol, Imidazol, Chinolin, Chinoxalin, 1,2-Dimethylimidazol, 1,3-Dimethylimidazolium-methylsulfat, steht, oder weiterhin
für ein Sulfoniumion steht, oder weiterhin
für ein Magnesium-Halogen-Kation steht,
n für die Zahl 1 oder 2 steht,
umsetzt
und mit Verbindungen der Formel (III) 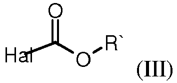 in welcher R' die oben angegebenen Bedeutungen haben und Hal für Halogen steht,
weiter zu Verbindungen der Formel (IV) 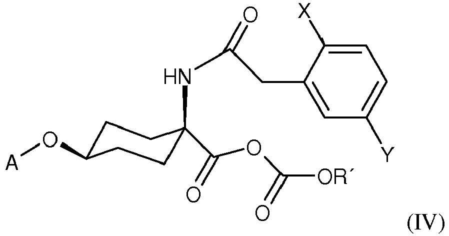 in welcher X, Y, A und R' die oben genannten Bedeutungen haben, umsetzt und
die weiter zu Verbindungen der Formel (IX) 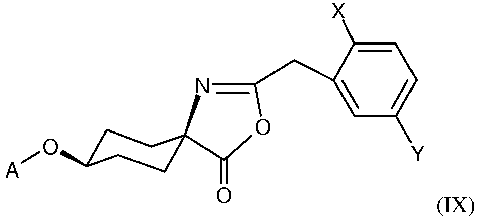 in welcher X, Y und A die oben angegebenen Bedeutungen haben, cyclisieren,
und diese wiederum
mit Verbindungen der Formel (V)
R"-OH (V)
in welcher
R" die oben angegebenen Bedeutungen haben
umsetzt.

2. Verfahren gemäß Anspruch 1, wobei
X für Chlor, Brom, Methyl, Ethyl, Propyl, Trifluormethyl, Methoxy, Difluormethoxy oder Trifluormethoxy steht,
Y für Wasserstoff, Chlor, Brom, Methoxy, Methyl, Ethyl, Propyl, Trifluormethyl oder Trifluormethoxy steht, wobei nur einer der Reste X oder Y für Trifluormethyl, Difluormethoxy oder Trifluormethoxy stehen darf,
A für C₁-C₆-Alkyl steht,
Hal für Chlor, Brom Fluor, Iod steht,
R' für C₁-C₆-Alkyl steht,
R" für C₁-C₆-Alkyl steht.

3. Verfahren gemäß Anspruch 1, wobei
X für Chlor, Brom, Methyl, Ethyl, Methoxy, Trifluormethyl, Trifluormethoxy oder Difluormethoxy steht,
Y für Chlor, Brom, Methyl, Ethyl, Propyl, Methoxy, Trifluormethyl oder Trifluormethoxy steht, wobei nur einer der Reste X oder Y für Trifluormethyl, Trifluormethoxy oder Difluormethoxy stehen darf,
A für C₁-C₄-Alkyl steht,
Hal für Chlor, Brom oder Fluor steht,
R' für C₁-C₄-Alkyl steht,
R" für C₁-C₄-Alkyl steht.

4. Verfahren gemäß Anspruch 1, wobei
X für Chlor, Brom, Methyl oder Trifluormethyl steht,
Y für Chlor, Brom oder Methyl steht,
A für Methyl, Ethyl, Propyl, Butyl oder Isobutyl steht,
Hal für Chlor oder Brom steht,
R' für Methyl, Ethyl, Propyl, Butyl oder Isobutyl steht,
R" für Methyl, Ethyl, Propyl, Butyl oder Isobutyl steht.

5. Verfahren gemäß Anspruch 1, wobei
X für Methyl steht,
Y für Methyl steht,
A für Methyl steht,
Hal für Chlor steht,
R' für Methyl oder Ethyl steht,
R" für Methyl oder Ethyl steht.

6. Verfahren gemäß Anspruch 1, wobei
X für Methyl steht,
Y für Methyl steht,
A für Methyl steht,
Hal für Chlor steht,
R' für Ethyl steht,
R" für Methyl steht.

7. Verfahren gemäß der Ansprüche 1, 2, 3, 4, 5 oder 6, wobei
M für Lithium, Natrium, Kalium, Caesium, Magnesium, Calcium oder für ein Ammoniumion steht, bei dem gegebenenfalls ein, zwei, drei oder alle vier Wasserstoffatome durch gleiche oder verschiedene Reste aus den Gruppen Wasserstoff C₁-C₅-Alkyl, C₁-C₅-Isoalkyl oder C₃-C₇-Cycloalkyl, die jeweils ein- oder mehrfach mit Fluor, Chlor, Brom, Cyano, Hydroxy substituiert sein können, ersetzt sein können und
n für die Zahl 1 oder 2 steht.

8. Verfahren gemäß der Ansprüche 1, 2, 3, 4, 5 oder 6, wobei
M für Lithium, Natrium, Kalium, Caesium, Magnesium oder Calcium und
n für die Zahl 1 oder 2 steht.

9. Verfahren gemäß der Ansprüche 1, 2, 3, 4, 5 oder 6, wobei
M für Lithium, Natrium, Kalium oder Caesium und
n für die Zahl 1 steht.

10. Verfahren gemäß der Ansprüche 1, 2, 3, 4, 5 oder 6, wobei
M für Natrium und
n für die Zahl 1 steht.

11. Verfahren gemäß Anspruch 1, wobei als Base Natrimcarbonat eingesetzt wird.

12. Verfahren gemäß Anspruch 1, wobei als Base Natriummethylat eingesetzt wird.

13. Verfahren gemäß Anspruch 1, wobei als Base Natriumhydroxid eingesetzt wird.

14. Verbindungen der Formel (IIb) 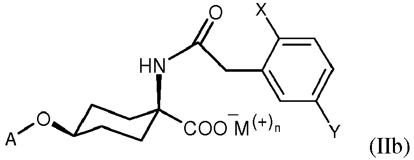 in welcher X, Y, A, M und n die oben angegebenen Bedeutungen haben.

15. Verbindungen der Formel (IV) 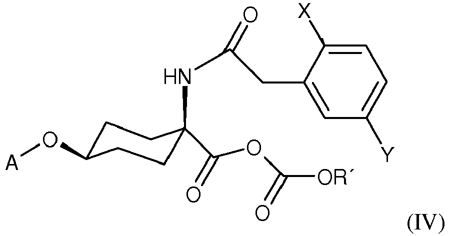 in welcher X, Y, A und R' die Bedeutungen gemäß Anspruch 1 haben.

16. Verbindungen der Formel (IX) 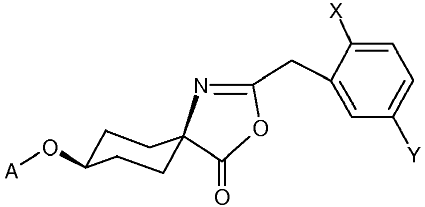 in welcher X, Y und A die Bedeutungen gemäß Anspruch 1 haben.

17. Verfahren zur Herstellung von Verbindungen der Formel (I') 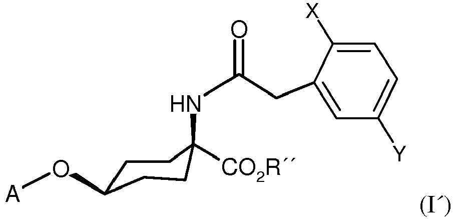 in welcher X, Y, A und R" die oben angegebenen Bedeutungen haben, **dadurch gekennzeichnet, dass** man zunächst
Verbindungen der Formel (IIa) 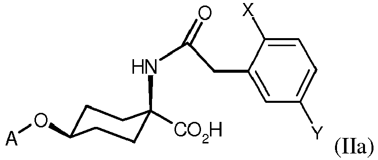 in welcher X, Y und A die oben genannten Bedeutungen haben
in Gegenwart einer Base zu Verbindungen der Formel (IIb) 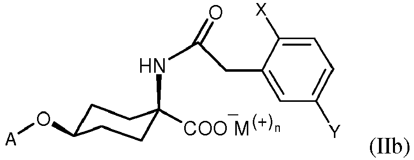 in welcher X, Y, A, M und n die oben genannten Bedeutungen haben, umsetzt
und mit Verbindungen der Formel (III) 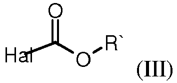 in welcher R' die oben angegebenen Bedeutungen haben
und Hal für Halogen steht,
weiter zu Verbindungen der Formel (IV) 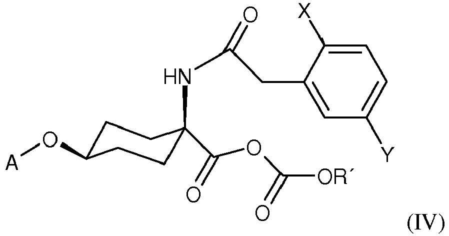 in welcher X, Y, A und R' die oben genannten Bedeutungen haben, umsetzt und
die weiter zu Verbindungen der Formel (IX) 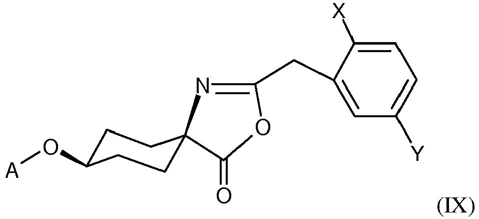 in welcher X, Y und A die oben angegebenen Bedeutungen haben, cyclisieren,
und diese wiederum
mit Verbindungen der Formel (V)
R"-OH (V)
in welcher
R" die oben angegebenen Bedeutungen haben umsetzt, wobei R' = R".

18. Verfahren zur Herstellung von Verbindungen der Formel (X) 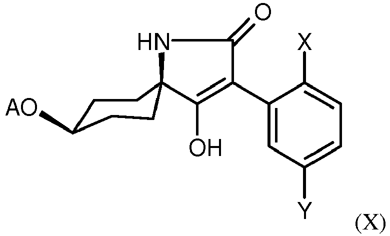 in welcher A, X und Y die oben angegebenen Bedeutungen haben, **dadurch gekennzeichnet, dass** man in einer Eintopfreaktion Verbindungen der Formel (IIa) 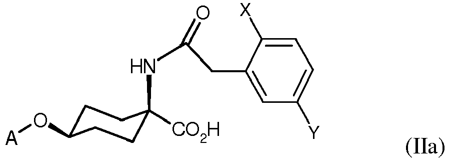 in welcher X, Y und A die oben genannten Bedeutungen haben,
in Gegenwart einer Base
zu Verbindungen der Formel (IIb) 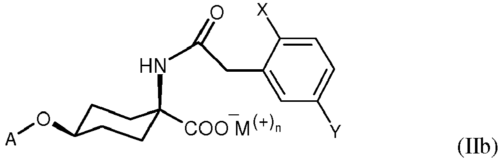 in welcher X, Y, A, M und n die oben genannten Bedeutungen haben umsetzt
und mit Verbindungen der Formel (III) 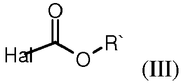 in welcher R' die oben angegebenen Bedeutungen haben und Hal für Halogen steht, in Gegenwart einer starken Base
ZLR" (XI)
wobei
Z für ein Alkalimetallion steht,
L für Sauerstoff oder Schwefel steht,
R" die oben angegebenen Bedeutungen hat,
umsetzt.

19. Verfahren gemäß Anspruch 18, wobei als Base und starke Base Natriummethylat eingesetzt wird

## Claims

1. Process for preparing compounds of the formula (I) 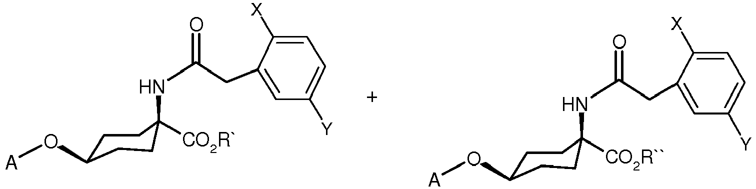 (I) in which
X represents alkyl, halogen, alkoxy, haloalkyl or haloalkoxy,
Y represents hydrogen, alkyl, alkoxy, halogen, haloalkyl or haloalkoxy, where only one of the radicals X or Y may represent haloalkyl or haloalkoxy,
A represents C₁-C₆-alkyl,
R' represents alkyl,
R" represents alkyl,
**characterized in that** initially compounds of the formula (IIa) 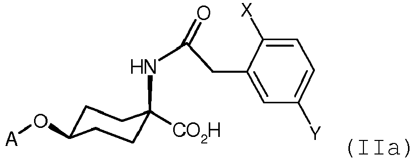 in which X, Y and A have the meanings mentioned above
are converted in the presence of a base into compounds of the formula (IIb) 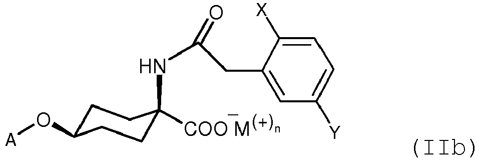 in which X, Y and A have the meanings mentioned above and
M represents an alkali metal ion, an ion equivalent of an alkaline earth metal, an ion equivalent of aluminium or an ion equivalent of a transition metal or furthermore represents an ammonium ion where optionally one, two, three or all four hydrogen atoms may be replaced by identical or different radicals from the groups C₁-C₅-alkyl, C₁-C₅-isoalkyl or C₃-C₇-cycloalkyl, each of which may be mono- or polysubstituted by fluorine, chlorine, bromine, cyano, hydroxy or interrupted by one or more oxygen or sulphur atoms, or furthermore represents a cyclic secondary or tertiary aliphatic or heteroaliphatic ammonium ion, for example morpholinium, thiomorpholinium, piperidinium, pyrrolidinium, or in each case protonated 1,4-diazabicyclo[2.2.2]octane (DABCO) or 1,5-diazabicyclo[4.3.0]undec-7-ene (DBU), or furthermore
represents a heterocyclic ammonium cation, for example in each case protonated pyridine, 2-methylpyridine, 3-methylpyridine, 4-methylpyridine, 2,4-dimethylpyridine, 2,5-dimethylpyridine, 2,6-dimethylpyridine, 5-ethyl-2-methylpyridine, pyrrole, imidazole, quinoline, quinoxaline, 1,2-dimethylimidazole, 1,3-dimethylimidazolium methylsulphate, or furthermore represents a sulphonium ion, or furthermore represents a magnesium halide cation,
n represents the number 1 or 2,
and reacted further with compounds of the formula (III) 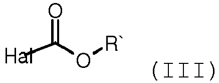 in which R' has the meanings given above and Hal represents halogen,
to give compounds of the formula (IV) 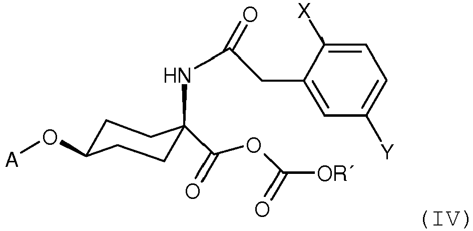 in which X, Y, A and R' have the meanings given above,
and
these are further cyclized to compounds of the formula (IX) 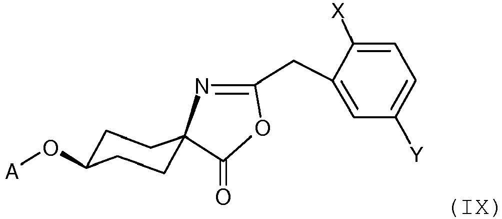 in which X, Y and A have the meanings given above, and these for their part are reacted
with compounds of the formula (V)
R"-OH (V)
in which
R" has the meanings given above.

2. Process according to Claim 1, where
X represents chlorine, bromine, methyl, ethyl, propyl, trifluoromethyl, methoxy, difluoromethoxy or trifluoromethoxy,
Y represents hydrogen, chlorine, bromine, methoxy, methyl, ethyl, propyl, trifluoromethyl or trifluoromethoxy, where only one of the radicals X or Y may represent trifluoromethyl, difluoromethoxy or trifluoromethoxy,
A represents C₁-C₆-alkyl,
Hal represents chlorine, bromine, fluorine, iodine,
R' represents C₁-C₆-alkyl,
R" represents C₁-C₆-alkyl.

3. Process according to Claim 1, where
X represents chlorine, bromine, methyl, ethyl, methoxy, trifluoromethyl, trifluoromethoxy or difluoromethoxy,
Y represents chlorine, bromine, methyl, ethyl, propyl, methoxy, trifluoromethyl or trifluoromethoxy, where only one of the radicals X or Y may represent trifluoromethyl, trifluoromethoxy or difluoromethoxy,
A represents C₁-C₄-alkyl,
Hal represents chlorine, bromine or fluorine,
R' represents C₁-C₄-alkyl,
R" represents C₁-C₄-alkyl.

4. Process according to Claim 1, where
X represents chlorine, bromine, methyl or trifluoromethyl,
Y represents chlorine, bromine or methyl,
A represents methyl, ethyl, propyl, butyl or isobutyl,
Hal represents chlorine or bromine,
R' represents methyl, ethyl, propyl, butyl or isobutyl,
R" represents methyl, ethyl, propyl, butyl or isobutyl.

5. Process according to Claim 1, where
X represents methyl,
Y represents methyl,
A represents methyl,
Hal represents chlorine,
R' represents methyl or ethyl,
R" represents methyl or ethyl.

6. Process according to Claim 1, where X represents methyl,
Y represents methyl,
A represents methyl,
Hal represents chlorine,
R' represents ethyl,
R" represents methyl.

7. Process according to any of Claims 1, 2, 3, 4, 5 or 6, where
M represents lithium, sodium, potassium, caesium, magnesium, calcium or represents an ammonium ion in which optionally one, two, three or all four hydrogen atoms may be replaced by identical or different radicals from the groups hydrogen C₁-C₅-alkyl, C₁-C₅-isoalkyl or C₃-C₇-cycloalkyl, each of which may be mono- or polysubstituted by fluorine, chlorine, bromine, cyano, hydroxy and
n represents the number 1 or 2.

8. Process according to any of Claims 1, 2, 3, 4, 5 or 6, where
M represents lithium, sodium, potassium, caesium, magnesium or calcium and
n represents the number 1 or 2.

9. Process according to any of Claims 1, 2, 3, 4, 5 or 6, where
M represents lithium, sodium, potassium or caesium and
n represents the number 1.

10. Process according to any of Claims 1, 2, 3, 4, 5 or 6, where
M represents sodium and
n represents the number 1.

11. Process according to Claim 1, where the base employed is sodium carbonate.

12. Process according to Claim 1, where the base employed is sodium methoxide.

13. Process according to Claim 1, where the base employed is sodium hydroxide.

14. Compounds of the formula (IIb) 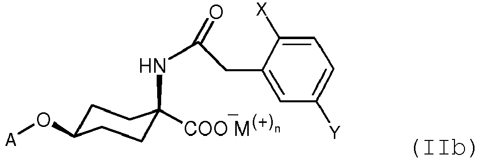 in which X, Y, A, M and n have the meanings given above.

15. Compounds of the formula (IV) 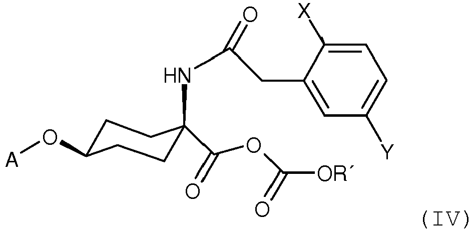 in which X, Y, A and R' have the meanings according to Claim 1.

16. Compounds of the formula (IX) 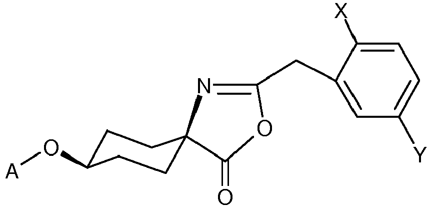 in which X, Y and A have the meanings according to Claim 1.

17. Process for preparing compounds of the formula (I') 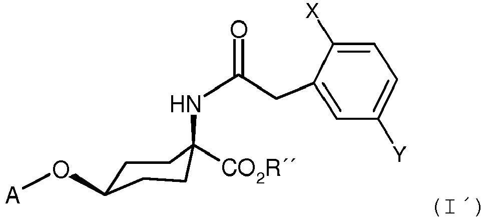 in which X, Y, A and R" have the meanings given above,
**characterized in that** initially compounds of the formula (IIa) 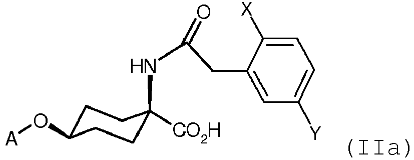 in which X, Y and A have the meanings mentioned above
are converted in the presence of a base into compounds of the formula (IIb) 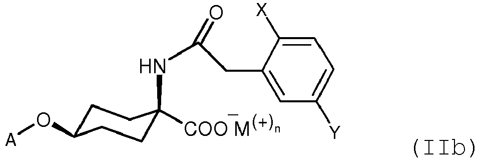 in which X, Y, A, M and n have the meanings mentioned above,
and reacted further with compounds of the formula (III) 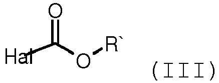 in which R' has the meanings given above and Hal represents halogen,
to give compounds of the formula (IV) 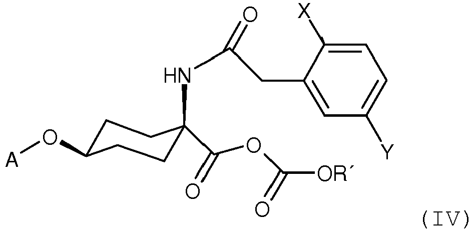 in which X, Y, A and R' have the meanings mentioned above,
and
these are further cyclized to compounds of the formula (IX) 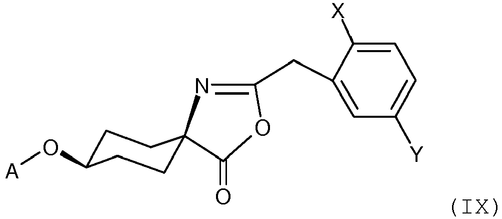 in which X, Y and A have the meanings given above, and these for their part are reacted with compounds of the formula (V)
R"-OH (V)
in which
R" has the meanings given above, where R' = R".

18. Process for preparing compounds of the formula (X) 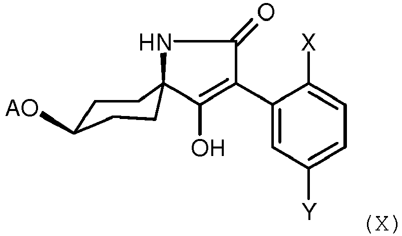 in which
A, X and Y have the meanings given above, **characterized in that**, in a one-pot reaction, compounds of the formula (IIa) 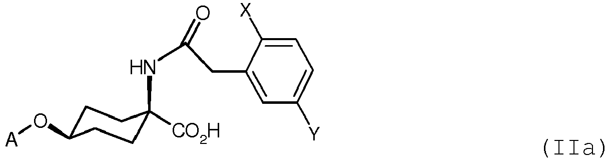 in which X, Y and A have the meanings mentioned above,
are converted in the presence of a base into compounds of the formula (IIb) 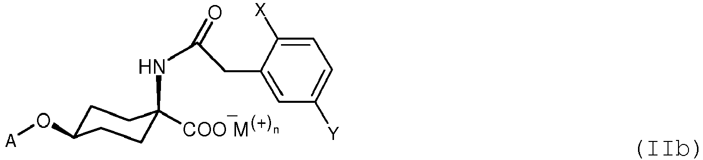 in which X, Y, A, M and n have the meanings mentioned above
and reacted with compounds of the formula (III) 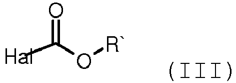 in which R' has the meanings given above and Hal represents halogen,
in the presence of a strong base
ZLR" (XI)
where
Z represents an alkali metal ion,
L represents oxygen or sulfur,
R " has the meanings given above.

19. Process according to Claim 18, where the base and strong base used is sodium methoxide.

## Revendications

1. Procédé de fabrication de composés de formule (I) 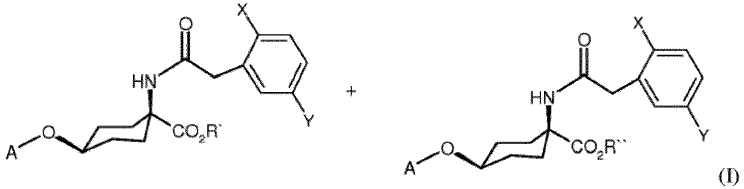 dans laquelle
X représente alkyle, halogène, alcoxy, halogénoalkyle ou halogénoalcoxy,
Y représente hydrogène, alkyle, alcoxy, halogène, halogénoalkyle ou halogénoalcoxy, seul un des radicaux X ou Y pouvant représenter halogénoalkyle ou halogénoalcoxy,
A représente alkyle en C₁-C₆,
R' représente alkyle,
R" représente alkyle,
**caractérisé en ce que** tout d'abord
des composés de formule (IIa) 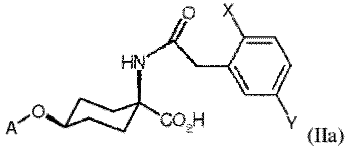 dans laquelle X, Y et A ont les significations indiquées précédemment,
sont mis en réaction en présence d'une base pour former des composés de formule (IIb) 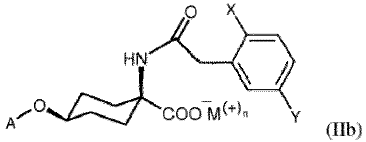 dans laquelle X, Y et A ont les significations indiquées précédemment, et
M représente un ion de métal alcalin, un équivalent d'ion d'un métal alcalino-terreux, un équivalent d'ion d'aluminium ou un équivalent d'ion d'un métal de transition, ou également
représente un ion ammonium, dans lequel un, deux, trois ou les quatre atomes d'hydrogène sont éventuellement remplacés par des radicaux identiques ou différents du groupe constitué par alkyle en C₁-C₅, isoalkyle en C₁-C₅ ou cycloalkyle en C₃-C₇, qui peuvent chacun être substitués une ou plusieurs fois avec fluor, chlore, brome, cyano, hydroxy, ou interrompus par un ou plusieurs atomes d'oxygène ou de soufre, ou également représente un ion ammonium cyclique secondaire ou tertiaire, aliphatique ou hétéroaliphatique, par exemple morpholinium, thiomorpholinium, pipéridinium, pyrrolidinium, ou 1,4-diazabicyclo[2.2.2]octane (DABCO) ou 1,5-diazabicyclo[4.3.0]undéc-7-ène (DBU), chacun protoné, ou également
représente un cation ammonium hétérocyclique, par exemple pyridine, 2-méthylpyridine, 3-méthylpyridine, 4-méthylpyridine, 2,4-diméthylpyridine, 2,5-diméthylpyridine, 2,6-diméthylpyridine, 5-éthyl-2-méthylpyridine, pyrrole, imidazole, quinoline, quinoxaline, 1,2-diméthylimidazole, méthylsulfate de 1,3-diméthylimidazolium, chacun protoné, ou également représente un ion sulfonium, ou également représente un cation magnésium-halogène,
n représente le nombre 1 ou 2,
puis mis en réaction avec des composés de formule (III) 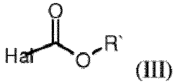 dans laquelle R' les significations indiquées précédemment,
et Hal représente halogène,
pour former des composés de formule (IV) 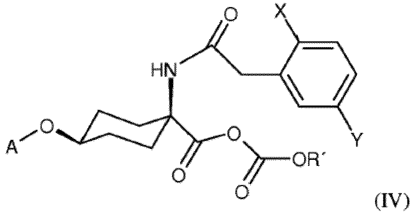 dans laquelle X, Y, A et R' ont les significations indiquées précédemment,
qui sont ensuite cyclisés en composés de formule (IX) 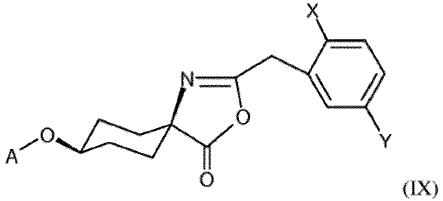 dans laquelle X, Y et A ont les significations indiquées précédemment,
et ceux-ci sont à leur tour mis en réaction avec des composés de formule (V)
R''-HO (V)
dans laquelle
R" a les significations indiquées précédemment.

2. Procédé selon la revendication 1, dans lequel
X représente chlore, brome, méthyle, éthyle, propyle, trifluorométhyle, méthoxy, difluorométhoxy ou trifluorométhoxy,
Y représente hydrogène, chlore, brome, méthoxy, méthyle, éthyle, propyle, trifluorométhyle ou trifluorométhoxy, uniquement un des radicaux X ou Y pouvant représenter trifluorométhyle, difluorométhoxy ou trifluorométhoxy,
A représente alkyle en C₁-C₆,
Hal représente chlore, brome, fluor, iode,
R' représente alkyle en C₁-C₆,
R" représente alkyle en C₁-C₆.

3. Procédé selon la revendication 1, dans lequel
X représente chlore, brome, méthyle, éthyle, méthoxy, trifluorométhyle, trifluorométhoxy ou difluorométhoxy,
Y représente chlore, brome, méthyle, éthyle, propyle, méthoxy, trifluorométhyle ou trifluorométhoxy, uniquement un des radicaux X ou Y pouvant représenter trifluorométhyle, trifluorométhoxy ou difluorométhoxy,
A représente alkyle en C₁-C₄,
Hal représente chlore, brome ou fluor,
R' représente alkyle en C₁-C₄,
R'' représente alkyle en C₁-C₄.

4. Procédé selon la revendication 1, dans lequel X représente chlore, brome, méthyle ou trifluorométhyle,
Y représente chlore, brome ou méthyle,
A représente méthyle, éthyle, propyle, butyle ou isobutyle,
Hal représente chlore ou brome,
R' représente méthyle, éthyle, propyle, butyle ou isobutyle,
R" représente méthyle, éthyle, propyle, butyle ou isobutyle.

5. Procédé selon la revendication 1, dans lequel
X représente méthyle,
Y représente méthyle,
A représente méthyle,
Hal représente chlore,
R' représente méthyle ou éthyle,
R" représente méthyle ou éthyle.

6. Procédé selon la revendication 1, dans lequel
X représente méthyle,
Y représente méthyle,
A représente méthyle,
Hal représente chlore,
R' représente éthyle,
R" représente méthyle.

7. Procédé selon les revendications 1, 2, 3, 4, 5 ou 6, dans lequel
M représente lithium, sodium, potassium, césium, magnésium, calcium ou un ion ammonium dans lequel un, deux, trois ou les quatre atomes d'hydrogène sont éventuellement remplacés par des radicaux identiques ou différents du groupe constitué par hydrogène, alkyle en C₁-C₅, isoalkyle en C₁-C₅ ou cycloalkyle en C₃-C₇, qui peuvent chacun être substitués une ou plusieurs fois avec fluor, chlore, brome, cyano, hydroxy, et
n représente le nombre 1 ou 2.

8. Procédé selon les revendications 1, 2, 3, 4, 5 ou 6, dans lequel
M représente lithium, sodium, potassium, césium, magnésium ou calcium, et
n représente le nombre 1 ou 2.

9. Procédé selon les revendications 1, 2, 3, 4, 5 ou 6, dans lequel
M représente lithium, sodium, potassium ou césium, et
n représente le nombre 1.

10. Procédé selon les revendications 1, 2, 3, 4, 5 ou 6, dans lequel
M représente sodium, et
n représente le nombre 1.

11. Procédé selon la revendication 1, dans lequel du carbonate de sodium est utilisé en tant que base.

12. Procédé selon la revendication 1, dans lequel du méthylate de sodium est utilisé en tant que base.

13. Procédé selon la revendication 1, dans lequel de l'hydroxyde de sodium est utilisé en tant que base.

14. Composés de formule (IIb) 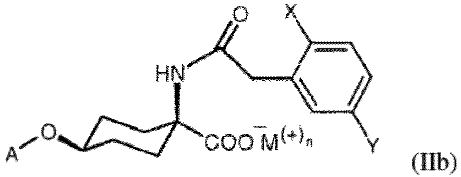 dans laquelle X, Y, A, M et n ont les significations indiquées précédemment.

15. Composés de formule (IV) 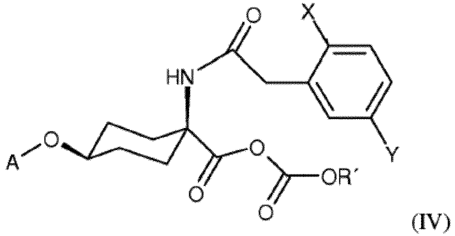 dans laquelle X, Y, A et R' ont les significations selon la revendication 1.

16. Composés de formule (IX) 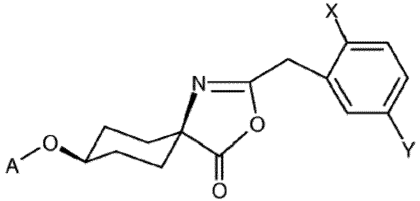 dans laquelle X, Y et A ont les significations selon la revendication 1.

17. Procédé de fabrication de composés de formule (I') 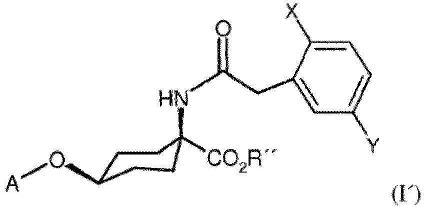 dans laquelle X, Y, A et R" ont les significations indiquées précédemment,
**caractérisé en ce que** tout d'abord
des composés de formule (IIa) 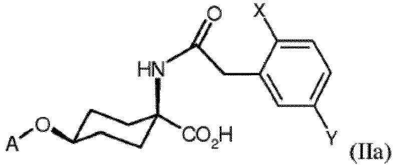 dans laquelle X, Y et A ont les significations indiquées précédemment,
sont mis en réaction en présence d'une base pour former des composés de formule (IIb) 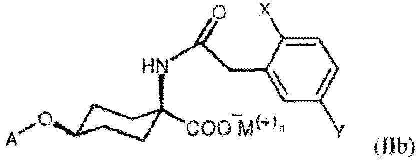 dans laquelle X, Y, A, M et n ont les significations indiquées précédemment,
puis avec des composés de formule (III) 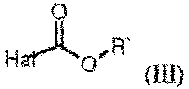 dans laquelle R' a les significations indiquées précédemment,
et Hal représente halogène,
pour former des composés de formule (IV) 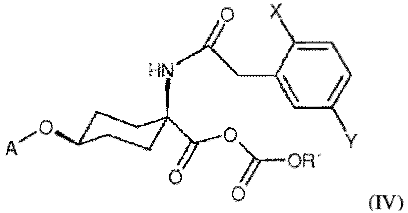 dans laquelle X, Y, A et R' ont les significations indiquées précédemment,
qui sont ensuite cyclisés en composés de formule (IX) 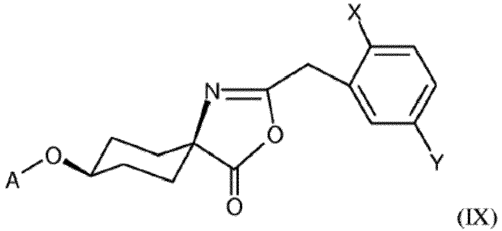 dans laquelle X, Y et A ont les significations indiquées précédemment,
et ceux-ci sont à leur tour mis en réaction avec des composés de formule (V)
R''-HO (V)
dans laquelle
R" a les significations indiquées précédemment, avec R' = R".

18. Procédé de fabrication de composés de formule (X) 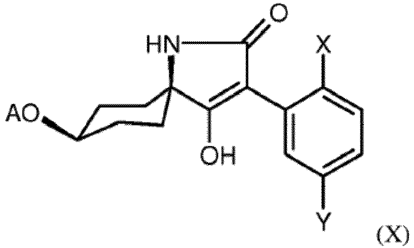 dans laquelle
A, X et Y ont les significations indiquées précédemment,
**caractérisé en ce que**, dans une réaction monotope,
des composés de formule (IIa) 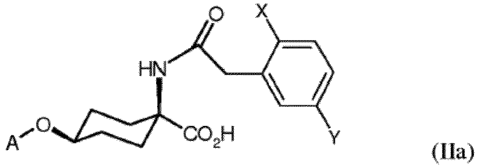 dans laquelle X, Y et A ont les significations indiquées précédemment,
sont mis en réaction en présence d'une base pour former des composés de formule (IIb) 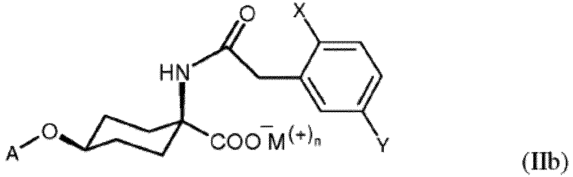 dans laquelle X, Y, A, M et n ont les significations indiquées précédemment,
puis avec des composés de formule (III) 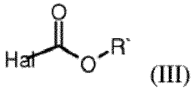 dans laquelle R' a les significations indiquées précédemment et Hal représente halogène,
en présence d'une base forte
ZLR'' (XI)
Z représentant un ion de métal alcalin,
L représentant l'oxygène ou le soufre,
R" ayant les significations indiquées précédemment.

19. Procédé selon la revendication 18, dans lequel du méthylate de sodium est utilisé en tant que base et base forte.
